# EUROPEAN PATENT SPECIFICATION

(11) **EP 1 767 162 B1**
(45) Date of publication and mention of the grant of the patent: **24.02.2010**
(21) Application number: 06019768.8
(22) Date of filing: 21.09.2006
(51) Int. Cl.: A61B 18/00

(54) **Method and system for treating pain during an electrosurgical procedure**
Verfahren und System, um Schmerz während eines electrochirurgischen Verfahrens zu behandeln
Methode et système de traitement de la douleur pendant une procédure électrochirurgicale

(30) Priority: 21.09.2005 US 232171
(43) Date of publication of application: 28.03.2007
(73) Proprietor: Covidien AG, 8212 Neuhausen am Rheinfall (CH)
(72) Inventor: Podhajsky, Ronald J., Boulder Colorado 80301 (US)
(74) Representative: HOFFMANN EITLE

(56) References cited:
- EP-A2- 1 070 518
- WO-A-00/67846
- US-A1- 2003 018 247
- US-A1- 2005 113 824

## Description

### BACKGROUND

The present disclosure is directed to control systems for electrosurgical generators, and more particularly, the present disclosure relates to a control system for electrosurgical generators and method for treating pain resulting from an electrosurgical procedure.

### TECHNICAL FIELD

Electrosurgical generators are employed by surgeons in conjunction with an electrosurgical instrument to cut, coagulate, desiccate, ablate and/or seal patient tissue. High frequency electrical energy, e.g., radio frequency (RF) energy, is produced by the electrosurgical generator and applied to the tissue by the electrosurgical tool. Both monopolar and bipolar configurations are commonly used during electrosurgical procedures. US 2005/0113824 discloses an electrosurgical generator and an electrosurgical instrument. The pre-amble of the independent claims is based on this document. EP 1 769 320 discloses an ablation apparatus and system to limit nerve conduction. This document forms state of the art according to Article 54 (3) EPC.

Electrosurgical techniques and instruments can be used to coagulate small diameter blood vessels or to seal large diameter vessels or tissue, e.g., soft tissue structures, such as lung, brain, skin, liver and intestine. A surgeon can ablate, cut, cauterize, coagulate, desiccate and/or simply reduce or slow bleeding, by controlling the intensity, frequency and duration of the electrosurgical energy applied between electrodes of an electrosurgical instrument and through the tissue. The energy involved in such procedures is sufficient to destroy designated tissue segments.

Such electrosurgical tissue-destroying procedures generally involve post-operative pain, caused by inflammation in close proximity to surviving peripheral nerves at the surgical site. There are two types of pain, nociceptive and neuropathic. Nociceptive pain is caused by injury to tissue in proximity to the peripheral nerves while neuropathic pain is caused by direct injury to the peripheral nerves. These peripheral nerves may survive the injury and regenerate overtime but some injuring events are severe and acute enough to leave an imprint on the memory circuits of the nervous system.

Post-operative pain following electrosurgical procedures is a combination of these types of pain. There have been attempts to relieve the pain caused by electrosurgical procedures. U.S. Patent Nos. 5,433,739, and 5,571,147, both issued to Sluijter et al.. and describe the application of electrosurgical energy via an electrode to an intermediate structure, namely an intervertebral disc, for the primary purpose of treating pain. The applied energy destroys innervation related to the disc, including neural structures remote from the position of the electrode for eliminating pain related to that innervation. Due to structural characteristics of the disc, heat applied to the disc is spread quickly and effectively to the periphery of the disc, beyond which the heat is sinked away rapidly. Also described is the application of other thermal treatments to the disc, for the primary purpose of destroying innervation related to the disc, including cryogenic cooling.

U.S. Patent Nos. 5,983,141, 6,161,048, 6,246,912, 6,259,952, all issued to Sluijter et al., describe a method for treating pain including direct application of energy via an electrode to a neural structure (or adjacent the neural structure) for the primary function of altering a function of the neural structure, and preventing lethal temperature elevation of the neural tissue during application of the energy. Modified waveforms having duty cycles are used in order to prevent thermal damage, particularly with long "off times and short "on" times.

However, the pain relieving procedures disclosed in the above-discussed patents are designed to relieve pain and not to prevent it. Furthermore, these procedures do not provide any treatment beyond modifying neural tissue function using electrical stimuli after the nerves have suffered damage. Thus, a need exists to provide a control system for an electrosurgical generator which controls generation and/or modulation of electrosurgical energy for providing pain modulation treatment in conjunction with the electrosurgical procedure thereby preventing pain. There is a further need for the pain modulation treatment to include a precursor treatment for minimizing secondary pain due to the anticipated effects (e.g., inflammation) of the primary function of the electrosurgical procedure.

### SUMMARY

The invention is defined by the independent claims.

In accordance with the present disclosure, a system is provided for controlling an electrosurgical generator outputting electrosurgical energy. The system includes at least one processor; a procedure control module executable on the at least one processor for controlling the generator to output electrosurgical energy which is optimized for performing an electrosurgical procedure. The system further includes a pain modulation control module executable on the at least one processor for controlling the generator to output electrosurgical energy which is optimized for administering pain modulation treatment in conjunction with performing the electrosurgical procedure.

In another embodiment of the disclosure, a method is provided for controlling an electrosurgical generator outputting electrosurgical energy. The method includes the steps of (a) controlling the generator to output electrosurgical energy which is optimized for performing an electrosurgical procedure; and (b) controlling the generator to output electrosurgical energy which is optimized for administering pain modulation treatment in conjunction with performing the electrosurgical procedure.

In accordance with still another embodiment of the disclosure, a method is provided for controlling an electrosurgical generator outputting electrosurgical energy. The method includes the steps of controlling the generator to output electrosurgical energy for performing an electrosurgical procedure at a surgical site. Controlling the generator includes imposing a duty cycle on the electrosurgical energy for optimizing the output electrosurgical energy for administration of pain modulation treatment including alteration of neural tissue at or proximate to the surgical site or a site related to the surgical site.

In accordance with a further embodiment of the disclosure, a method for controlling an electrosurgical generator outputting electrosurgical energy is provided. The method includes the steps of providing for determining an operational mode selected from the group of operational modes consisting of an electrosurgical procedure mode, a combination mode and a pain modulation treatment mode; providing for controlling the generator for outputting electrosurgical energy optimized for performance of the electrosurgical procedure when the electrosurgical procedure mode is selected; and providing for controlling the generator for outputting electrosurgical energy optimized for pain modulation treatment when the pain modulation treatment mode is selected.

The method further includes the steps of providing for determining, when the combined mode is selected, at least one pain modulation surgical mode selected from the group consisting of: a pre-surgical mode, a concurrent-with-surgery mode and a post-surgery mode; providing for controlling the generator for outputting electrosurgical energy optimized for pre-surgical pain modulation treatment when the pre-surgical mode is selected; and providing for controlling the generator for outputting electrosurgical energy optimized for performance of the electrosurgical procedure when the concurrent-with-surgery mode is not selected. The method finally includes the steps of providing for controlling the generator when the concurrent-with-surgery mode is selected for combining outputting electrosurgical energy optimized for performance of the electrosurgical procedure and outputting electrosurgical energy that is optimized for pain modulation treatment; and providing for controlling the generator to output electrosurgical energy that is optimized for post-surgical pain management treatment when the post-surgical mode is selected.

According to another aspect of the present disclosure, there is provided a control system for controlling a radio-frequency (RF) generator outputting RF energy. The system has at least one processor and a procedure control module executable on the at least one processor for controlling the generator to output electrosurgical energy which is optimized for performing an electrosurgical procedure. The system further has a pain modulation control module executable on the at least one processor for controlling the generator to output electrosurgical energy which is optimized for administering pain modulation treatment in conjunction with performing the electrosurgical procedure.

### BRIEF DESCRIPTION OF THE FIGURES

Various embodiments will be described herein below with reference to the drawings wherein:
FIG. 1 is a schematic diagram of an electrosurgical system in accordance with the present disclosure;
FIG. 2 is a block diagram of a control system of the electrosurgical system shown in FIG. 1;
FIGS. 3-6 are modulated frequency signals output by the electrosurgical system in accordance with the present disclosure; and
FIG. 7 is a flowchart of a method for controlling an electrosurgical generator in accordance with the present disclosure.

### DETAILED DESCRIPTION

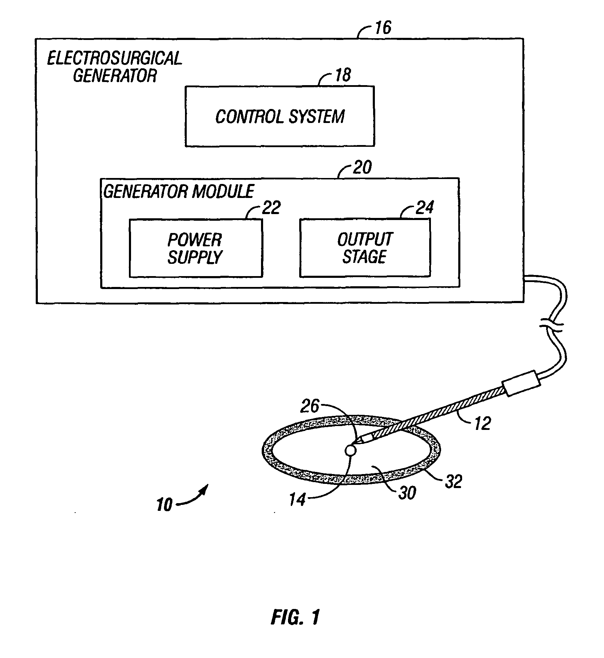

Reference should be made to the drawings where like reference numerals refer to similar elements throughout the various figures. Referring to FIG. 1, there is shown a schematic diagram of one embodiment of the presently-disclosed electro-surgical system 10 having an electrosurgical instrument 12 for delivering electrosurgical energy to a patient at a surgical site 14. An electrosurgical generator 16 is provided having a generator module 20 for generating electrosurgical energy and a control system 18 for controlling the generator module 20, where the electrosurgical energy output by the generator module 20 is modulated by the control system 18. The modulated electrosurgical energy is provided by the generator 16 to the electrosurgical instrument 12.

It is known that the particular waveform of electrosurgical energy can be modulated to enhance a desired surgical effect, e.g., cutting, coagulating, sealing, blending, ablating, etc. For example, in a "cutting" mode, an uninterrupted sinusoidal waveform in the frequency range of 100 kHz to 4MHz with a crest factor in the range of 1.4 to 2.0 is used. In a "blend" mode, a sinusoidal waveform with a duty cycle in the range of 25% to 75% and a crest factor in the range of 2.0 to 5.0 is used. In a "coagulate" mode, a sinusoidal waveform with a duty cycle of approximately 10% or less and a crest factor in the range of 5.0 to 12.0 is used.

The present disclosure also provides for a secondary modulated pulse in addition to the first modulated pulse (e.g., the electrosurgical pulse) that is not lethal to the nerve tissue and is above a threshold of stimulation so that the memory circuit is reprogrammed. This allows the nerve tissue to remember a less painful sensation thereby alleviating the pain sensation.

The present disclosure provides for a system having a first function and a second function. The first function provides for a voltage waveform that is suitable to heat a tissue above a temperature of sixty-five degrees to alter a function of the tissue. The second function provides for another discrete function that mediates a post-operative pain to the patient. The second function provides for a stimulation of a tissue by a physiological relevant signal. The signal is not lethal to the tissue and does not heat the tissue to any temperatures above sixty five degrees Celsius. The signal, instead, is above a threshold of stimulation of the tissue and treats the tissue to reduce a post operative pain sensation of the patient. The signal, in one embodiment, may have a peak voltage that is sufficient to stimulate the nerve tissue with a duty cycle that is sufficient to prevent the nerve tissue from heating to an average temperature level that will destroy the nerve tissue.

It has been observed that when enough energy is deposited in the tissues, an irreversible change to the tissue occurs. Sensory peripheral nerves have cell bodies or soma. The soma is located in the dorsal root ganglion close to the spinal cord. These cell bodies also have axons that communicate with the dorsal part of the spinal cord. The cell bodies also have axons that traverse out to the far reaches of the extremities (arms, legs, etc.). Some of these sensory nerves are stimulated by an injury to tissues that are closely adjacent to their innervation site. This is known as nociceptive pain. By contrast, neuropathic pain is caused by a direct injury to their axons.

Peripheral nerves may, in fact, survive injury to their axons, however an acute injury event may be severe enough to leave an imprint on the so called "memory circuits" of the nervous system. The present disclose provides a threshold stimulation to a portion of the nervous system so the so called "memory circuits" do not transmit pain post operatively. The threshold stimulation of the present disclosure is a physiological relevant signal to mediate post-operative pain. Advantageously, such as threshold stimulation is generated as a feature of the generator.

Referring back to FIG. 1, a typical electrosurgical instrument 12 includes an end-effector 26 having appropriate structures for affecting tissue, such as grasping, dissecting and/or clamping tissue. The end-effector 26 may further include at least one delivery device, such as an electrode, for delivering the electrosurgical energy to the patient. Mechanical action, such as clamping, may be used by the electrosurgical instrument 12 in addition to the application of electrosurgical energy to obtain a surgical effect. As can be appreciated, the electrode(s) may be configured as monopolar, bipolar or macro-bipolar. Further, the electrosurgical instrument 12 may be configured as suitable for performing endoscopic or open surgery.

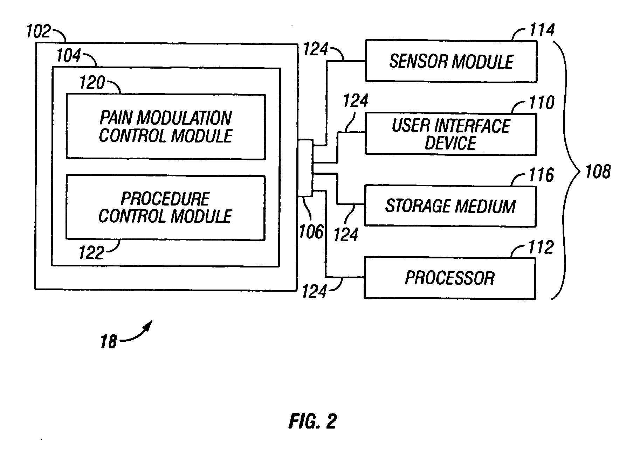

With reference to FIG. 2, the control system 18 is shown, where the control system 18 includes at least one processor 102 having a control module 104 executable on the at least one processor 102, and at least one input/output (I/O) port 106 for communicating with at least one peripheral device 108. The at least one peripheral device 108 may include, for example, at least one of at least one user interface device 110, at least one processor 112, a sensor module 114 having at least one sensor, and/or at least one storage medium 116. At least a portion of information and/or signals input to the at least one processor 102 are processed by the control module 104 which outputs control signals for modulating the electrosurgical energy in accordance with the input information and/or signals. The control system 18 is coupled to the generator module 20 by connections that may include wired and/or wireless connections for providing the control signals to the generator module 20.

The control module 104 further includes pain modulation control module 120 for modulating the electrosurgical energy output by the electrosurgical generator 16, such as by generating control signals for determining parameter settings of the generator, which may include parameter settings of the generator module 20. The electrosurgical energy is modulated by the pain modulation control module 120 for optimizing the electrosurgical energy for administering pain modulation treatment in conjunction with the electrosurgical procedure being performed.

The control module 104 further includes a procedure control module 122 for modulating the electrosurgical energy output by the electrosurgical generator 16, such as by generating control signals for determining parameter settings of the generator 16, which may include parameter settings of the generator module 20. The electrosurgical energy is modulated by the procedure control module 122 for optimizing the electrosurgical energy for performing the electrosurgical procedure. Control signals generated by the pain modulation control module 120 override or modify control signals generated by the procedure control module 122, or vice versa, in accordance with design choice and depending on procedural factors, such as the stage of the electrosurgical procedure and pain modulation being performed. It is contemplated that the pain modulation module 120 may control the generator 16 to output a different bandwidth for the pain modulation treatment than is output for the electrosurgical procedure.

With further reference to FIG. 1, typical electrosurgical generator module 20 includes a power supply 22 for generating energy and an output stage 24 which modulates the energy. The power supply 22 generates energy, such as RF, microwave, ultrasound, infrared, ultraviolet, laser or thermal energy. In one embodiment, the power supply 22 is a high voltage DC or AC power supply for producing electrosurgical current, where control signals received from the control module 104 control parameters of the electrosurgical energy output by the power supply 22, including the magnitude of the voltage and current of the electrosurgical energy. The output stage 24 modulates the output energy and its effective energy, such as via a waveform generator, where control signals received from the control module 104 control waveform parameters of the electrosurgical energy output by the output stage 24, e.g., frequency, waveform shape, pulse width, duty cycle, crest factor, and/or repetition rate.

It is also contemplated that the generator 16 may be connected, e.g., via a network, such as the internet, to a remote off-site server and/or database providing information, such as instrument operating information, mappings, algorithms and/or programs. Updated information may be provided on a regular basis and downloaded to the generator 16 as needed and/or prior to surgery. As can be appreciated, this enables the user to obtain updated information regarding operation of the instrument, electrical parameters, and ideal curves for optimizing pain modulation. In addition, this also enables the generator manufacturer to provide updated information on a regular basis. It is also contemplated that the user may be able to receive diagnostics remotely in this fashion relating to the instruments and/or generators being utilized, either on demand by the user, prior to an operation or automatically during a scheduled download.

With further reference to FIG. 2, the control module 104 regulates the generator 16, e.g., the power supply 22 and/or the output stage 24, according to at least a portion of information and/or signals input to the at least one processor 102 by the peripheral device(s) 108. The electrosurgical system 10 may be controlled by the control module 104 to operate in a selected operational mode selected from an electrosurgical mode for performing an electrosurgical procedure without providing pain modulation treatment; a combined mode for providing pain modulation in conjunction with an electrosurgical procedure; or a pain modulation mode for providing pain modulation treatment without performing an electrosurgical procedure. Mode selection may be performed by a user, a peripheral processor or by the control module 104. The peripheral device(s) 108 are coupled to the at least one processor 102 via a wired or wireless communication interface 124 to allow input of pre-surgical information, to enter information, instructions, requests, mode selection and/or to control various parameters of the electrosurgical energy delivered to the patient during the electrosurgical procedure. Parameters of the delivered electrosurgical energy which may be regulated by the pain modulation control module 120 and/or the procedure control module 122 include, for example, voltage, current, resistance, intensity, power, frequency, amplitude, and/or waveform parameters, e.g., waveform shape, pulse width, duty cycle, crest factor, and/or repetition rate of the output and/or effective energy.

Pre-surgical data may include patient data, e.g., age, weight, present medical condition, etc., and/or data describing the surgical procedure, e.g., location of surgical site, extent of surgical action (e.g., with respect to time and space), type of pain management desired (e.g., extent with respect to time and space, including pre-surgery, concurrent-with-surgery and/or post-surgery), electrosurgical instrument being used, the type of electrosurgical procedure to be performed, operating conditions (pressure applied by electrosurgical instrument, gap between electrodes, etc.), desired surgical results and/or the tissue type upon which the electrosurgical procedure is being performed. A recognition technology may be employed to relay instrument parameters to the control module 104, e.g., a smart system, such as described in commonly owned U.S. Patent Publication No. 2005-0113818 entitled "CONNECTOR SYSTEMS FOR ELECTROSURGICAL GENERATOR" The control module 104 may be designed to automatically set specific parameters of the generator 16 based upon the input information. One system for controlling a medical generator in accordance with user entered pre-surgical information is described in commonly owned U.S. Patent Publication No. 2004-0015163, entitled "METHOD AND SYSTEM FOR CONTROLLING OUTPUT OF RF MEDICAL GENERATOR". Additional pre-surgical data may be obtained by administering an initialization pulse of electrosurgical energy to the patient and sensing or measuring responses in the patient to the initialization pulse.

The control module 104 preferably includes software instructions executable by the at least one processor 102 for processing data received by the peripheral device(s) 108, and for outputting control signals to the generator 16. The software instructions may be stored in a storage medium such as a memory internal to the at least one processor 102 and/or a memory accessible by the at least one processor 102, such as an external memory, e.g., an external hard drive, floppy diskette, CD-ROM, etc. Control signals from the control module 104 for controlling the generator 16 may be converted to analog signals by a digital-to-analog converter (DAC) which may be included in the at least one processor 102 or external thereto. It is contemplated that the at least one processor 102 may include circuitry, e.g., analog devices, for processing information input by the peripheral device(s) 108 and determining the control signals to be generated for controlling the generator 16, e.g., for adjusting parameter settings of the generator 16. Further, an audio or visual feedback monitor or indicator (not shown) may be employed to convey information to the surgeon regarding the status of a component of the electrosurgical system or the electrosurgical procedure.

Control signals for controlling the generator 16 are determined by performing algorithms and processing pre-surgical data and/or information entered by the peripheral device(s) 108. Furthermore, the control signals may be determined by accessing further information and/or desired values, such as by accessing a knowledge base and/or consulting a mapping (e.g., an ideal curve, look-up-table, etc.) stored by or accessible by the control module 104. Control signals generated by the procedure control module 122 determine parameter settings for performing the electrosurgical procedure. Control signals generated by the pain modulation control module 120 determine parameter settings to be used for pain modulation, which modify or override the parameter settings when the pain modulation is in effect when operating in the combined mode. Furthermore, when operating in the combined mode one or more of the following pain modulation surgical modes may be selected for modulating the electrosurgical energy for providing pain modulation treatment: a pre-surgery mode for providing pain modulation treatment before beginning an electrosurgical procedure; a concurrent-with-surgery mode for providing pain modulation treatment during the electrosurgical procedure, and/or a post-surgery mode for providing pain modulation treatment after completion of the electrosurgical procedure.

When the pre-surgery mode is selected, the pain modulation control module 120 determines control signals for controlling the generator 16 for initializing parameter settings thereof for outputting modified electrosurgical energy for a selected time interval prior to beginning the electrosurgical procedure.

Initialization of the parameters of the generator 16 for beginning the electrosurgical procedure is performed by the procedure control module 122 when the concurrent-with-surgery mode is not selected, and is performed by the pain modulation control module 120 and the procedure control module 122 when the concurrent-with-surgery mode is selected.

In the case where control signals for adjusting parameter settings of the generator 16 are being determined by both the pain modulation control module 120 and the procedure control module 122, the pain modulation control module 120 decides which of the respective determinations of the control modules 120, 122 takes precedence, or how to combine the respective determinations for determining the control signals. For example, the control signals or selected individual control signals may be set in accordance with a selected combination technique, such as averaging the determinations (with or without weighting either of the determinations), performing an algorithm (e.g., logic function(s) and/or computational function(s)) on the respective determinations, etc., or a combination thereof.

When the concurrent-with-surgery mode is selected, the parameter settings of the generator 16 are optimized during the electrosurgical procedure for pain modulation and performance of the electrosurgical procedure substantially simultaneously in accordance with control signals generated by the pain modulation control module 120 and the procedure control module 122. In a first sub-mode of the concurrent-with-surgery mode, a first combination technique is used in which at least one function is executed by the pain modulation control module 120 for controlling the generator by performing at least one function for adjusting parameter settings of the generator, wherein the adjusted parameter settings are a function of settings optimized for administering the electrosurgical procedure and settings optimized for administering the pain modulation treatment when the first sub-mode is selected. The function may include imposing at least one waveform parameter optimized for pain modulation on one or more waveform parameters optimized for the electrosurgical procedure. For example, a duty cycle optimized for the pain modulation may be imposed on the waveform parameters optimized for the electrosurgical procedure. The function or parameters of the function may be selectable, such as by a user.

In a second sub-mode, a second combination technique is used in which the pain modulation control module 120 controls the generator for alternating adjustment of the parameter setting of the generator 16 between settings optimized for the pain modulation and settings that are optimized for performance of the electrosurgical procedure. Accordingly, the pain modulation is provided intermittently during the procedure, e.g., interlaced with administration of electrosurgical energy that is optimized for administering the electrosurgical procedure. Timing parameters of the alternations may be selectable, such as by a user. In a third sub-mode, the first and second sub-modes are combined.

When the post-surgery mode is selected, the pain modulation control module 120 determines control signals for adjusting parameter settings of the generator 16 for providing the post-surgery pain modulation following the electrosurgical procedure. The post-surgery treatment may be provided immediately following the surgery or after a delay, where the delay may be determined manually or automatically by the pain modulation control module 122. The pain modulation post-surgery treatment may be provided after an extended period (hours, days, etc.) by operating in the pain modulation mode. The control signals may be determined by using the pre-surgical data and/or by using updated information entered by the peripheral device(s) 108 during and/or following the procedure. Accordingly, any combination of pre-surgery, concurrent-with-surgery and post-surgery pain modulation may be provided.

With further reference to FIGS. 1 and 2, pain associated with an electrosurgical procedure may include secondary pain which occurs due to a physiological response (e.g., inflammation, nociceptive pain and neuropathic pain) to the electrosurgical procedure. Nerves affected by the physiological response may be located at the surgical site 14, at an adjacent area 30 near the surgical site, or at an area 32 peripheral to area 30 and remote from the surgical site 14. For example, in an ablation procedure in which a strong inflammatory response occurs, pain is experienced due to nociceptive pain and neuropathic pain in close proximity to surviving nerves, and central sensitization may occur. In another example, application of continual electrosurgical energy stimulates nerves which may cause a perception of pain.

Pain treatment is described in U.S. Patent Nos. 5,433,739, 5,571,147, 5,983,141, 6,161,048, 6,246,912, 6,259,952, all issued to Sluijter et al.. A technique which has not been described in the above mentioned patents is to treat pain with an instrument having a primary purpose of performing an electrosurgical procedure, where the pain modulation treatment is provided in conjunction with the electrosurgical procedure, including at least one of pre-surgery, concurrent-with-surgery or post-surgery. Also not described is the treatment of anticipated secondary pain as a precursor treatment in anticipation of the development of inflammatory pain due to an electrosurgical procedure. Furthermore, a control system is not described which controls an electrosurgical system to selectively operate in an electrosurgical mode, combination mode and a pain modulation mode.

Application of pulsed electrosurgical energy has the effect of destroying, altering or desensitizing nerves to which the pulses are delivered, where parameters of the energy and its waveform determine how the nerves are affected. Accordingly, when providing pain modulation, the pain modulation control module 120 determines the control signals that are provided by the control module 104 to the electrosurgical generator 16 for adjusting parameter settings of the electrosurgical generator and the output energy for imposing a duty cycle on a continuous wave. Resulting effective energy of the output electrosurgical energy includes a pulse train having a duty cycle which is less than 100%.

The pain modulation control module 120 modulates the electrosurgical energy for administering pain modulation treatment in conjunction with the electrosurgical procedure, which may be provided pre-surgery, concurrent-with-surgery and/or post-surgery. The electrosurgical energy output for performing the surgical procedure may be pulsed or continuous. The pain modulation control module 120 modulates the electrosurgical energy by imposing a duty cycle thereto or by modifying characteristics of existing pulsing for optimization for pain modulation. The pain modulation control module 120 may further modify the electrosurgical energy so that it is not optimized for either just the electrosurgical procedure or the pain modulation, but where a combination technique is used for striking a compromise for achieving both the surgical effect and the pain modulation effect.

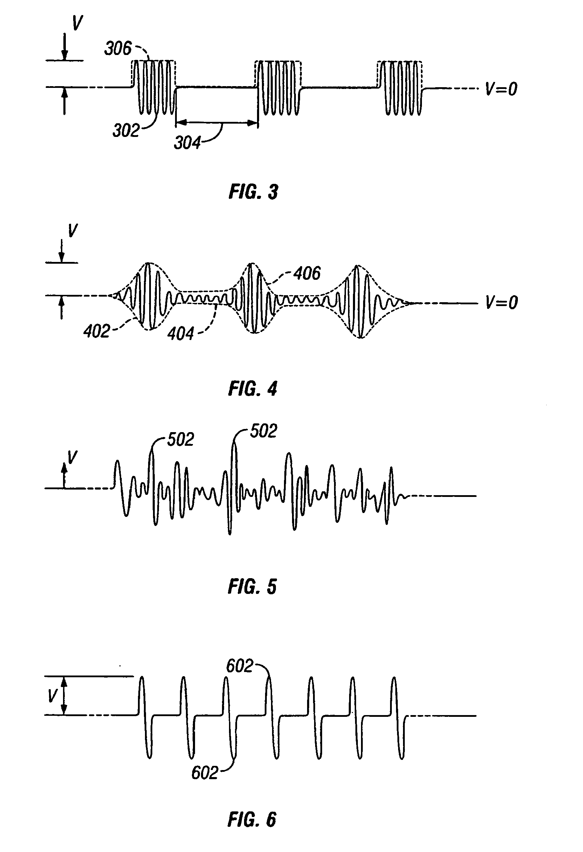

Examples of pulse trains of modified waveforms effective in altering neural function for pain modulation are shown in FIGS. 3-6, where a pulse from one pulse train is interposed periodically in the pulse train of another. More simply put, a pain modulating pulse is interspersed within a cutting, ablation, blend waveform.

FIG. 3 shows a burst of high frequency RF oscillations 302 followed by an interval 304 of steady low voltage (V=0). An envelope represented by dotted lines 306 defines pulses of the effective energy of the modified waveform shown in FIG. 3 as perceived by the patient's body, in which each burst of oscillations defines a pulse.

FIG. 4 shows another variation of a pulse train in which intervals 402 having high peak voltage swings and intervals 404 having low peak voltage swings having a reduced average power are delivered to the patient. An envelope represented by dotted lines 406 defines pulses of the effective energy of the modified waveform shown in FIG. 4 as perceived by the patient's body. The baseline voltage is shown at zero. Such a modulation envelope 406 can be achieved by selecting parameters of the generator 16, such as signal modulation in the output stage 24 for providing low frequency filtering, or for varying the input or output gain of a high frequency signal output by the power supply 22.

FIG. 5 shows yet another embodiment of an interrupted high frequency waveform or pulse train having a non-periodic variation of voltage. The maxima point 502, which is perceived by the patient's body as a pulse of effective energy, can occur at random positions in time. The time difference between maxima can also vary in an irregular or even random way. This waveform may have no repeating or periodic structure but may be analogous to high frequency noise with random amplitudes, peaks, zero crossings, and carrier high frequencies. Such a waveform can be generated by random noise generators, spark gap signals, or other noisy signals that are known in the field of signal generation. Furthermore, filtering can be applied in a wave generator and power amplifier of the output stage 24, so that lower frequencies in the physiologic range will not be present to give undesirable stimulation effects.

FIG. 6 shows yet another possible high frequency waveform of interrupted, repeated bipolar pulses 602 having a frequency, for example, in a physiologic stimulation frequency range (i.e., 0 to about 300 Hertz). The pulse on-time in accordance with the effective energy as perceived by the patient's body may be low enough so that the power deposition can be kept low enough to prevent heating, and yet the peak voltage V is enough to alter the neural function.

Variations of such waveforms are possible with the same intermittent high frequency effect for pain on neurological modification. For instance, a baseline V=0 may not pertain and a slowly varying baseline of non-zero value can be used. The time average of the signal need not be zero. The on and off switching of a high frequency signal, such as in FIG. 3, can be done at a non-periodic or non-regular, repeating rate so that, on average, the polarization effects in the tissue are still maintained at a low level. The average power deposition can still be maintained at a low level with non-periodic, interrupted high frequency waveforms. The high frequency carrier frequency may also be non-constant. Varying or combined or superposed high frequency waveforms can be used as the carriers, and these combined or composite high frequency waveforms can be interrupted or modulated in accordance with the present system and disclosure. Pulse waveforms with high frequency carriers can be shaped in a variety of ways, for example with fast rising leading edges and slow or falling off or exponential trailing edges. The signal generator waveform can have a peak intensity which is much higher than the average or RMS intensity to yield a high electromagnetic field or current density on the neural tissue while maintaining the average power deposition in the tissue at a sufficiently low level to prevent heating above lethal tissue temperatures (viz. 40 to 50 degrees Celsius).

It is contemplated that the rising edges of pulses of the effective energy's pulse train (as perceived by the patient's body) have the greatest effect in altering nerve function. Accordingly, it is possible that pulse frequency is proportional to achievement of nerve function alteration, and that the desired type and level of pain modulation may be achieved by adjusting the pulse frequency. Amplitude may be decreased proportional to increases in pulse frequency for preventing undesired surgical effects, such as when pain modulation is being administered non-simultaneously with the electrosurgical energy (e.g., pre-surgery, post-surgery or in-between intervals of application of electrosurgical energy). In addition, it is contemplated that the "on" and "off' times of individual pulses is selectable by adjusting the parameter settings. For example, a longer "on" time relative to the "off' time may be selected when pain modulation is provided during a procedure, such as a coagulation procedure, for increasing the surgical effect while providing the pain modulation.

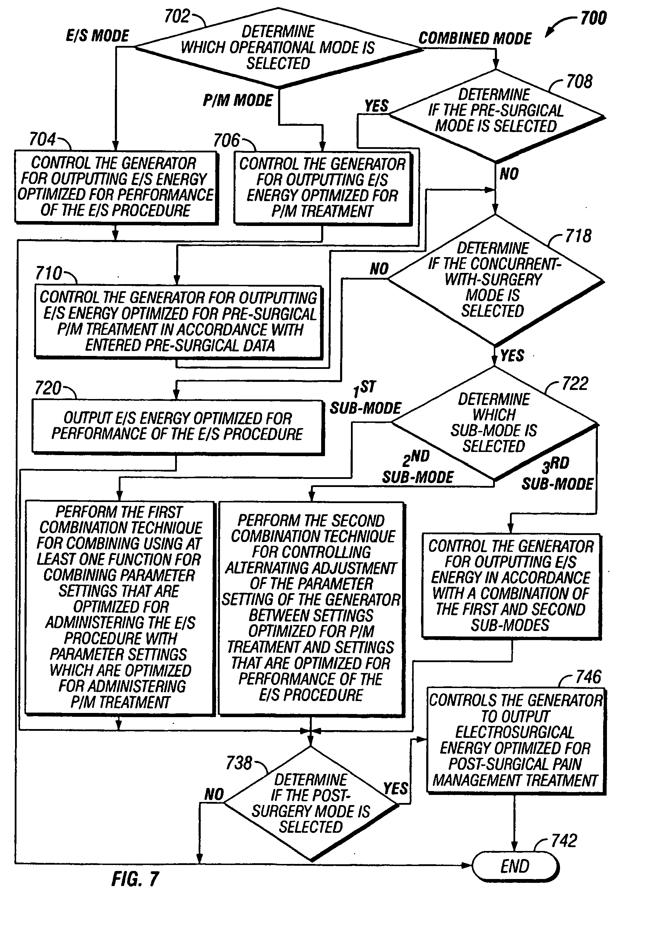

With respect to FIG. 7, a flowchart 700 of steps performed by the control module 104 is shown. At step 702, upon initiation of a procedure, a determination is made as to which operational mode is selected, e.g., which of the electrosurgical (e/s) procedure mode, combination mode or pain modulation (p/m) treatment mode is selected. If the electrosurgical procedure mode is selected, at step 704, the control module 104 controls the generator 16 for outputting electrosurgical energy optimized for performance of the electrosurgical procedure. After the electrosurgical procedure is finished, the end step 742 is executed. If the pain modulation treatment mode is selected, at step 706, the control module 104 controls the generator 16 for outputting the electrosurgical energy optimized for pain modulation treatment. After the pain modulation treatment is finished, the end step 742 is executed. If the combined mode is selected, at step 708, the control module 104 determines if the pre-surgical mode is selected. If so, at step 710, the control module 104 controls the generator 16 for outputting electrosurgical energy optimized for pre-surgical pain modulation treatment, e.g., in accordance with entered pre-surgical data. If not, step 710 is skipped and step 718 is performed. At step 718, the control module 104 determines if the concurrent-with-surgery mode is selected.

If not, at step 720, the control module controls the generator 16 for outputting electrosurgical energy optimized for performance of the electrosurgical procedure. If so, at step 722, the control module determines if the first, second or third sub-mode is selected. If the first sub-mode is selected, at step 726, the control module 104 performs the first combination technique for combining, including controlling the generator by performing at least one function for adjusting parameter settings of the generator, wherein the adjusted parameter settings are a function of settings optimized for administering the electrosurgical procedure and settings optimized for administering the pain modulation treatment. The function may include imposing at least one waveform parameter optimized for pain modulation on one or more waveform parameters optimized for the electrosurgical procedure. For example, a duty cycle optimized for the pain modulation may be imposed on the waveform parameters optimized for the electrosurgical procedure.

If the second sub-mode is selected, at step 730, the control module 104 performs the second combination technique for controlling the generator for alternating adjustment of the parameter setting of the generator 16 between settings optimized for the pain modulation and settings that are optimized for performance of the electrosurgical procedure. If the third sub-mode is selected, at step 734, the control module 104 controls the generator 16 for outputting electrosurgical energy in accordance with a combination of the first and second sub-modes.

Upon completion of the electrosurgical procedure, at step 738, a determination is made if the post-surgery mode is selected. If not, end step 742 is executed. If so, at step 746, the control module 104 controls the generator 16 to output electrosurgical energy that is optimized for post-surgical pain management treatment. Following the completion of step 746, end step 742 is executed.

The following is a description of an exemplary procedure for ablating tissue using an appropriate electrosurgical instrument which has a pain modulation mode option. The ablation procedure without pain modulation would include application of electrosurgical RF energy consisting of an RF "on" cycle where a continuous RF carrier wave is administered and an RF "off" cycle with duration and amplitudes appropriate for the treated tissue. The control system 18 determines that the concurrent-with-surgery mode and the first sub-mode are selected for combining pain modulation treatment with performance of the ablation procedure. The first combination technique is used for imposing a duty cycle on the RF waveform used for the ablation procedure. For example, the electrosurgical generator 16 is controlled by the control system 104 to output RF energy having a repetition rate of 2 Hz and a duty cycle of 80%, where the effective RF energy is "high" or "on" for 400 msec and "low" or "off' for 100msec. The RF pulsed energy may be applied for the duration of the ablation procedure for ablating the tissue and simultaneously providing pain modulation, or for a portion of the ablation procedure, such as before, after or interspersed between application of continual electrosurgical energy for effecting the ablation.

A pain modulation mode option may be provided with a variety of electrosurgical instruments, such as electrosurgical pencils or other instruments designed for separating, coagulating, sealing, desiccating, etc. tissue. Via the user interface 110, an operator may select the pain modulation mode, which may include selecting pre-surgery, concurrent-with-surgery and/or post-surgery pain modulation. The parameter settings may be adjusted manually or automatically, including the timing of application of the parameter settings. Automatic adjustment of the parameter settings may be in accordance with the pre-surgical data and/or properties sensed during the procedure.

Use of the pain modulation mode may provide an immediate anesthetic effect at the surgical site as well as provide secondary pain modulation, including for nerves at or next to the surgical site, and nerves that are remote from the surgical site that are expected to be affected by post-operative nociceptive pain and neuropathic pain. For electrosurgical procedures in which a traditional anesthesia is administered, the secondary pain modulation will minimize pain experienced once the anesthesia wears off. The secondary pain modulation further minimizes central sensitization due to post-operative nociceptive pain and neuropathic pain. Duration of the effects of the pain modulation differ in accordance with factors, such as the type and degree of pain modulation administered, the parameter settings used during the pain modulation, the location and types of nerves affected by the electrosurgical procedure and the pain modulation. It is known under some circumstances for nerves to regain normal function with time after alteration, as well as for destroyed nerves to regenerate.

During an electrosurgical procedure which includes pain modulation treatment, adjustments may be made to the parameter settings manually or automatically, in accordance with observations made by the operator and/or results of sensing by the sensor module 114. Furthermore, intervals of pain modulation concurrent-with-surgery may be triggered manually or automatically by a predetermined event related to an observation and/or sensed property. Observations may include visual monitoring of contractions of muscle located proximate the nerves being treated, which may indicate the occurrence of stimulation of the nerves. A user may enter observation data to the control module 104 via peripheral devices 108 to input data indicative of observations made by the user.

Sensors of sensor module 114, such as sensors mounted on a secondary probe (e.g., needle), may be positioned at a location proximate nerves remote from the surgical site which may be affected by the electrosurgical procedure for monitoring those nerves or local tissue characteristics, such as physical or electrical properties (e.g., temperature, impedance, optical transmission properties, etc.). Neural monitoring and/or conductive velocity tests may be performed, as are known in the art. In addition, a stimulation mode having sensory and/or motor functions may be provided for use with the pain modulation mode for determining proximity to a nerve or nerve bundle. The simulation mode may include applying electrosurgical energy at a frequency and amplitude for stimulating a muscle and/or nerve for identifying the location of the muscle and/or nerve. Identification may be observed, perceived and reported by the patient and/or sensed by a sensing device. The type and degree of pain modulation administration may be decided automatically or manually in accordance with the results of such a proximity determination.

It is further contemplated that observation data and data output from the sensor module corresponding to sensed properties may be used for determining parameters of energy that will be delivered for administering pain modulation treatment. Sensed properties, such as tissue moisture content or impedance, can be helpful in calculating the degree to which energy delivered to the surgical site 14 will be diminished before arriving at the peripheral site 32. Accordingly, the amount of energy being delivered to the peripheral site 32 can be predicted in accordance with the amount of energy that is delivered to surgical site 14. Likewise, the energy delivered to surgical site 14 may be determined in accordance with the desired energy delivery to the peripheral site 14.

For an ablation procedure, once ablation occurs at the surgical site 14, the ablated tissue is no longer innervated and no longer has vascularity. The absence of vascularity leads to heat convection properties conducive to directing energy to the peripheral area 32. Accordingly, heat spreads effectively to nerves located in area 32, allowing for effective post-surgical treatment of those nerves in area 32 which are remote from the surgical site 14 for altering their function or ablating them as desired. By applying pain modulation treatment to the surgical site 14 as a post-surgical treatment, the nerves at peripheral site 32 which are most likely to be affected by secondary pain due to nociceptive pain and neuropathic pain are indirectly treated.

The system and method according to the present disclosure, which allows for interspersion of secondary pulses configured preventing pain, can be applied to other energy-based surgical and treatment system, such as laser, cryogenic, microwave, high intensity ultrasound, ultrasound, and the like. The present disclosure is further intended to encompass other secondary pain mediation treatments including laser, cryogenic, microwave, high intensity ultrasound, ultrasound subsequent to the surgical procedure.

## Claims

1. A control system (18) for controlling an electrosurgical generator (16) outputting electrosurgical energy comprising:
at least one processor (102);
a procedure control module (122) executable on the at least one processor configured to generate control signals to control the generator to output electrosurgical energy which is optimized for performing an ablation procedure; and
**characterised by**:
a pain modulation control module (120) executable on the at least one processor configured to generate control signals to control the generator to output electrosurgical energy, said pain modulation control module being optimized for administering a pain modulation treatment in conjunction with performing the ablation procedure, wherein the pain modulation control module is configured to generate control signals to control the generator to output electrosurgical energy that override or modify control signals generated by the procedure control module.

2. The control system according to Claim 1, configured such that the pain modulation control module controls the generator to impose a duty cycle on the electrosurgical energy for establishing a pulse train associated with effective energy of the output electrosurgical energy.

3. The control system according to Claim 1, configured such that control of the generator by the pain modulation control module in conjunction with performance of the electrosurgical procedure includes controlling the generator to output electrosurgical energy optimized for administration of pain modulation treatment at least one of before administration of the electrosurgical energy for performing the electrosurgical procedure, during administration of the electrosurgical energy for performing the electrosurgical procedure and after administration of the electrosurgical energy for performing the electrosurgical procedure.

4. The control system according to Claim 3, configured such that:
controlling the generator includes adjusting parameter settings of the generator; and
controlling the generator to output electrosurgical energy optimized for administering pain modulation treatment during administration of the electrosurgical energy for performing the electrosurgical procedure includes at least one of:
controlling the generator by the pain modulation control module to output electrosurgical energy which is optimized for administering pain modulation treatment intermittently with controlling the generator by the procedure control module to output electrosurgical energy which is optimized for administration during performance of an electrosurgical procedure; and
controlling the generator by the pain modulation control module and the procedure control module substantially simultaneously including adjusting the generator's parameter settings in accordance with at least one function for combining adjusting parameter settings of the generator to output electrosurgical energy which is optimized for administration during performance of an electrosurgical procedure with adjusting parameter settings of the generator to output electrosurgical energy which is optimized for administering pain modulation treatment.

5. The control system according to Claim 1, 2, 3 or 4, configured such that the at least one processor operates in a mode selectable from each of an electrosurgical mode in which the generator is controlled by the procedure control module; a pain modulation mode in which the generator is controlled by the pain modulation control module; and a combination mode, in which the generator is controlled by both the procedure control module and the pain modulation control module.

6. The control system according to any one of the preceding claims, configured such that
controlling the generator includes adjusting parameter settings of the generator;
the at least one processor receives input data for at least one of selecting the mode, entering pre-surgical data, entering observation data, and selecting parameter settings of the generator; and
at least a portion of the input data is used to determine adjustments to parameter settings of the generator.

7. The control system according to any one of the preceding claims, further comprising a sensor module having at least one sensor sensing properties at or proximate to a surgical site or a site related to the surgical site and generating corresponding sensing signals, wherein the pain modulation control module is configured to control the generator in accordance with at least a portion of the sensing signals.

8. The control system according to any one of the preceding claims, wherein the pain modulation treatment is configured to treat a site remote from a surgical site to which the electrosurgical energy is administered during performance of the electrosurgical procedure.

9. The control system according to any one of the preceding claims, wherein the pain modulation control module is configured such that pain modulation treatment includes treatment of anticipated secondary pain.

10. A control system according to Claim 9, said secondary pain treatment being selected from the group consisting of laser, cryogenic, microwave, high intensity ultrasound and ultrasound.

11. The control system according to any one of the preceding claims, configured such that controlling the generator includes adjusting parameter settings of the generator for adjusting parameters of the output electrosurgical energy, wherein parameters of the output electrosurgical energy include at least one of pulse frequency, carrier frequency, waveform shape, pulse width, duty cycle, crest factor, amplitude, amplitude modulation, a treatment overlay, and frequency modulation.

12. The control system according to Claim 3 or any claim dependent thereon, configured such that the pain modulation mode further includes a selectable stimulation mode having at least one of sensory and motor functions for determining proximity to a nerve or nerve bundle.

13. The control system according to any one of the preceding claims for controlling a radio-frequency (RF) generator outputting RF energy.

14. The control system according to any one of the preceding claims, wherein said pain modulation control treatment is suitable to modulate said output for stimulating a tissue for reducing an amount of a pain mechanism.

15. A method for controlling an electrosurgical generator (16) outputting electrosurgical energy, the method comprising the steps of:
(a) generating control signals, thereby controlling the generator to output electrosurgical energy which is optimized for performing an ablation procedure; and **characterised by**:
(b) generating control signals, thereby controlling the generator to output electrosurgical energy which is optimized for administering pain modulation treatment in conjunction with performing the ablation procedure,
wherein control signals generated by the step (b) override or modify control signals generated by step (a).

16. The method according to Claim 15, wherein step (b) includes controlling the generator for imposing a duty cycle on the electrosurgical energy for establishing a pulse train associated with effective energy of the output electrosurgical energy.

17. The method according to Claim 15, wherein step (b) includes controlling the generator to output electrosurgical energy optimized for administration of pain modulation treatment at least one of before administration of the electrosurgical energy for performing the electrosurgical procedure, during administration of the electrosurgical energy for performing the electrosurgical procedure and after administration of the electrosurgical energy for performing the electrosurgical procedure.

18. The method according to Claim 17, wherein:
controlling the generator includes adjusting parameter settings of the generator; and
controlling the generator to output electrosurgical energy optimized for administering pain modulation treatment during administration of electrosurgical energy for performing the electrosurgical procedure includes at least one of:
controlling the generator to output electrosurgical energy which is optimized for administering pain modulation treatment intermittently with controlling the generator to output electrosurgical energy which is optimized for administration during performance of an electrosurgical procedure; and
controlling the generator to output electrosurgical energy optimized for both administration of pain modulation treatment and performance of the electrosurgical procedure substantially simultaneously, including adjusting the generator's parameter settings in accordance with at least one function for combining adjusting parameter settings of the generator to output electrosurgical energy which is optimized for administration during performance of an electrosurgical procedure and adjusting parameter settings of the generator to output electrosurgical energy which is optimized for administering pain modulation treatment.

19. The method according to any one of Claims 15 to 18, wherein controlling the generator step includes
adjusting parameter settings of the generator;
receiving input data for at least one of entering pre-surgical data, entering observation data, and selecting parameter settings of the generator; and
adjusting parameter settings of the generator using at least a portion of the input data for determining the adjusting.

20. The method according to any one of Claims 15 to 19, the method further comprising the steps of:
receiving sensing signals corresponding to sensing of properties at or proximate to the surgical site or a site related to the surgical site;
wherein the controlling the generator to output electrosurgical energy optimized for administration of pain modulation treatment is performed in accordance with at least a portion of the sensing signals.

21. The method according to any one of Claims 15 to 20, wherein the administration of pain modulation treatment includes treatment of anticipated secondary pain.

22. The method according to any one of Claims 15 to 21, wherein controlling the generator includes adjusting parameter settings of the generator for adjusting parameters of the output electrosurgical energy, wherein parameters of the output electrosurgical energy include at least one of pulse frequency, repetition rate, waveform shape, pulse width, duty cycle, crest factor, and amplitude.

23. The method according to any one of Claims 15 to 22, wherein step (a) is performed until an area associated with the surgical site is ablated so that it no longer has vascularity, after which step (b) is performed at the surgical site, wherein due to the lack of vascularity of the area, heat caused by administration of the electrosurgical energy during step (a) is spread effectively to an area having vascularity which is peripheral to the area lacking vascularity for providing pain modulation treatment to nerves located at the peripheral area having vascularity.

24. The method according to any one of Claims 15 to 23 comprising the step of controlling the generator to output electrosurgical energy for performing an electrosurgical procedure at a surgical site including imposing a duty cycle on the electrosurgical energy for optimizing the output electrosurgical energy for administration of pain modulation treatment including alteration of neural tissue at a location in and around the surgical site.

## Patentansprüche

1. Steuerungssystem (18) zum Steuern eines elektrochirurgischen Generators (16), der elektrochirurgische Energie ausgibt, aufweisend:
zumindest einen Prozessor (102);
ein Verfahrenssteuerungsmodul (122), das auf dem zumindest einen Prozessor ausführbar ist, das eingerichtet ist, um Steuerungssignale zu erzeugen, um den Generator zu steuern, um elektrochirurgische Energie auszugeben, die zum Durchführen einer Ablationsprozedur optimiert ist; und
**gekennzeichnet durch**:
ein Schmerzmodulationssteuerungsmodul (120), das auf dem zumindest einen Prozessor ausführbar ist, das eingerichtet ist, um Steuerungssignale zu erzeugen, um den Generator zu steuern, um elektrochirurgische Energie auszugeben, wobei das Schmerzmodulationssteuerungsmodul zum Anwenden einer Schmerzmodulationsbehandlung in Verbindung mit dem Durchführen der Ablationsprozedur optimiert ist, wobei das Schmerzmodulationssteuerungsmodul eingerichtet ist, um Steuerungssignale zu erzeugen, um den Generator zu steuern, um elektrochirurgische Energie auszugeben, die Steuerungssignale, die **durch** das Verfahrenssteuerungsmodul erzeugt wurden, zu übersteuern oder zu modifizieren.

2. Steuerungssystem nach Anspruch 1, derart eingerichtet, dass das Schmerzmodulationssteuerungsmodul den Generator steuert, um der elektrochirurgischen Energie einen Arbeitszyklus aufzuerlegen, um einen Pulszug einzurichten, der mit effektiver Energie der ausgegebenen elektrochirurgischen Energie verbunden ist.

3. Steuerungssystem nach Anspruch 1, derart eingerichtet, dass ein Steuern des Generators durch das Schmerzmodulationssteuerungsmodul in Verbindung mit dem Durchführen des elektrochirurgischen Verfahrens ein Steuern des Generators enthält, um elektrochirurgische Energie auszugeben, die zum Anwenden einer Schmerzmodulationsbehandlung vor dem Anwenden der elektrochirurgischen Energie zum Durchführen des elektrochirurgischen Verfahrens und/oder während des Anwendens der elektrochirurgischen Energie zum Durchführen des elektrochirurgischen Verfahrens und/oder nach dem Anwenden der elektrochirurgischen Energie zum Durchführen des elektrochirurgischen Verfahrens optimiert ist.

4. Steuerungssystem nach Anspruch 3, derart eingerichtet, dass:
das Steuern des Generators ein Einstellen von Parametereinstellungen des Generators enthält; und
das Steuern des Generators, um elektrochirurgische Energie auszugeben, die zum Anwenden einer Schmerzmodulationsbehandlung während des Anwendens der elektrochirurgischen Energie zum Durchführen des elektrochirurgischen Verfahrens optimiert ist, zumindest eines der folgenden enthält:
ein Steuern des Generators durch das Schmerzmodulationssteuerungsmodul, um elektrochirurgische Energie auszugeben, die zum Anwenden einer Schmerzmodulationsbehandlung optimiert ist, intermittierend mit dem Steuern des Generators durch das Verfahrenssteuerungsmodul, um elektrochirurgische Energie auszugeben, die zum Anwenden während der Durchführung eines elektrochirurgischen Verfahrens optimiert ist; und
ein Steuern des Generators im Wesentlichen gleichzeitig durch das Schmerzmodulationssteuerungsmodul und dss Verfahrenssteuerungsmodul inklusive des Einstellens der Generatorparametereinstellungen in Übereinstimmung mit zumindest einer Funktion zum Kombinieren von Einstellungsparametereinstellungen des Generators, um elektrochirurgische Energie auszugeben, die zum Anwenden während der Durchführung eines elektrochirurgischen Verfahrens optimiert ist, mit Einstellungsparametereinstellungen des Generators, um elektrochirurgische Energie auszugeben, die zum Anwenden einer Schmerzmodulationsbehandlung optimiert ist.

5. Steuerungssystem nach Anspruch 1, 2, 3 oder 4, derart eingerichtet, dass der zumindest eine Prozessor in einem Modus betrieben wird, der aus jedem, einem elektrochirurgischen Modus, in dem der Generator durch das Verfahrenssteuerungsmodul gesteuert wird, einem Schmerzmodulationsmodus, in dem der Generator durch das Schmerzmodulationssteuerungsmodul gesteuert wird, und einem Kombinationsmodus, in dem der Generator sowohl durch das Verfahrenssteuerungsmodul als auch das Schmerzmodulationssteuerungsmodul gesteuert wird, auswählbar ist.

6. Steuerungssystem nach einem der vorgehenden Ansprüche, derart eingerichtet, dass
das Steuern des Generators ein Einstellen von Parametereinstellungen des Generators enthält; der zumindest eine Prozessor Eingabedaten für das Auswählen des Modus, das Eingeben von prä-chirurgischen Daten, das Eingeben von Beobachtungsdaten und/oder das Auswählen von Parametereinstellungen des Generators empfängt; und
zumindest ein Teil der Eingabedaten verwendet wird, um Einstellungen für Parametereinstellungen des Generators zu bestimmen.

7. Steuerungssystem nach einem der vorgehenden Ansprüche, ferner umfassend ein Sensormodul mit zumindest einem Sensor, der Eigenschaften an oder in der Nähe von einer chirurgischen Lokalisation oder einer mit der chirurgischen Lokalisation verbundenen Stelle erkennt und entsprechende Sensorsignale erzeugt, wobei das Schmerzmodulationssteuerungsmodul eingerichtet ist, um den Generator in Übereinstimmung mit zumindest einem Abschnitt der Sensorsignale zu steuern.

8. Steuerungssystem nach einem der vorgehenden Ansprüche, wobei die Schmerzmodulationsbehandlung eingerichtet ist, um eine Stelle entfernt von der chirurgischen Lokalisation, welcher die elektrochirurgische Energie während der Durchführung des elektrochirurgischen Verfahrens zugeführt wurde, zu behandeln.

9. Steuerungssystem nach einem der vorgehenden Ansprüche, wobei das Schmerzmodulationssteuerungsmodul derart eingerichtet ist, dass eine Schmerzmodulationsbehandlung eine Behandlung von antizipiertem Sekundärschmerz enthält.

10. Steuerungssystem nach Anspruch 9, wobei die Sekundärschmerzbehandlung aus der Gruppe ausgewählt ist, die aus Laser, Kryogen, Mikrowelle, Hochintensitätsultraschall und Ultraschall besteht.

11. Steuerungssystem nach einem der vorgehenden Ansprüche, derart eingerichtet, dass ein Steuern des Generators ein Einstellen von Parametereinstellungen des Generators zum Einstellen von Parametern der ausgegebenen elektrochirurgischen Energie enthält, wobei Parameter der ausgegebenen elektrochirurgischen Energie zumindest eine Pulsfrequenz, eine Trägerfrequenz, eine Wellenform, eine Pulsweite, einen Arbeitszyklus, einen Scheitelfaktor, eine Amplitude, eine Amplitudenmodulation, eine Behandlungsüberlagerung und/oder eine Frequenzmodulation enthalten.

12. Steuerungssystem nach Anspruch 3 oder einem davon abhängigen Anspruch, derart eingerichtet, dass der Schmerzmodulationsmodus ferner einen wählbaren Stimulationsmodus enthält, der Sensor- und/oder Motorfunktionen zum Bestimmen der Nähe zu einem Nerv oder einem Nervenbündel aufweist.

13. Steuerungssystem nach einem der vorgehenden Ansprüche zum Steuern eines Generators von Radiofrequenzen (RF), der RF-Energie ausgibt.

14. Steuerungssystem nach einem der vorgehenden Ansprüche, wobei die Schmerzmodulationssteuerungsbehandlung geeignet ist, um die Ausgabe zum Stimulieren eines Gewebes zu modulieren, um eine Menge eines Schmerzmechanismus zu reduzieren.

15. Verfahren zum Steuern eines elektrochirurgischen Generators (16), der elektrochirurgische Energie ausgibt, wobei das Verfahren die folgenden Schritte umfasst:
(a) Erzeugen von Steuerungssignalen, dadurch Steuern des Generators, um elektrochirurgische Energie auszugeben, die zum Durchführen eines Ablationsverfahrens optimiert ist; und **gekennzeichnet durch**:
(b) Erzeugen von Steuerungssignalen, **dadurch** Steuern des Generators, um elektrochirurgische Energie auszugeben, die zum Anwenden einer Schmerzmodulationsbehandlung in Verbindung mit dem Durchführen des Ablationsverfahrens optimiert ist,
wobei Steuerungssignale, die **durch** Schritt (b) erzeugt wurden, Steuerungssignale, die **durch** Schritt (a) erzeugt wurden, übersteuern oder modifizieren.

16. Verfahren nach Anspruch 15, wobei Schritt (b) ein Steuern des Generators zum Auferlegen eines Arbeitszyklus auf die elektrochirurgische Energie zum Erzeugen eines Pulszugs, der mit effektiver Energie der ausgegebenen elektrochirurgischen Energie verbunden ist, enthält.

17. Verfahren nach Anspruch 15, wobei Schritt (b) ein Steuern des Generators enthält, um elektrochirurgische Energie auszugeben, die zum Anwenden einer Schmerzmodulationsbehandlung vor dem Anwenden der elektrochirurgischen Energie zum Durchführen des elektrochirurgischen Verfahrens, während des Anwendens der elektrochirurgischen Energie zum Durchführen des elektrochirurgischen Verfahrens und/oder nach dem Anwenden der elektrochirurgischen Energie zum Durchführen des elektrochirurgischen Verfahrens optimiert ist.

18. Verfahren nach Anspruch 17, wobei:
das Steuern des Generators ein Einstellen von Einstellungsparametern des Generators enthält; und
das Steuern des Generators, um elektrochirurgische Energie auszugeben, die optimiert ist zum Anwenden einer Schmerzmodulationsbehandlung während des Anwendens elektrochirurgischer Energie zum Durchführen des elektrochirurgischen Verfahrens, zumindest eines der folgenden enthält:
ein Steuern des Generators, um elektrochirurgische Energie auszugeben, die optimiert ist zum Anwenden einer Schmerzmodulationsbehandlung, intermittierend mit einem Steuern des Generators, um elektrochirurgische Energie auszugeben, die optimiert ist zum Anwenden während des Durchführens eines elektrochirurgischen Verfahrens; und
ein Steuern des Generators, um elektrochirurgische Energie auszugeben, die für das im Wesentlichen gleichzeitige Anwenden sowohl einer Schmerzmodulationsbehandlung als auch das Durchführen des elektrochirurgischen Verfahrens optimiert ist, inklusive des Einstellens der Parametereinstellungen des Generators in Übereinstimmung mit einer Funktion zum Kombinieren des Einstellens von Parametereinstellungen des Generators, um elektrochirurgische Energie auszugeben, die optimiert ist zum Anwenden während des Durchführens eines elektrochirurgischen Verfahrens und/oder zum Einstellen von Parametereinstellungen des Generators, um elektrochirurgische Energie auszugeben, die zum Anwenden einer Schmerzmodulationsbehandlung optimiert ist.

19. Verfahren nach einem der Ansprüche 15 bis 18, wobei der Schritt des Steuerns des Generators
ein Einstellen von Parametereinstellungen des Generators;
ein Aufnehmen von Eingabedaten zum Eingeben von prä-chirurgischen Daten, Eingeben von Beobachtungsdaten und/oder Auswählen von Parametereinstellungen des Generators; und
ein Einstellen von Parametereinstellungen des Generators unter Verwendung zumindest eines Teils der Eingabedaten zum Bestimmen der Einstellung enthält.

20. Verfahren nach einem der Ansprüche 15 bis 19, wobei das Verfahren ferner die folgenden Schritte umfasst:
Aufnehmen von Sensorsignalen, die einem Erkennen von Eigenschaften an oder in der Nähe von der chirurgischen Lokalität oder einer mit der chirurgischen Lokalität verbundenen Stelle entsprechen;
wobei das Steuern des Generators, um elektrochirurgische Energie auszugeben, die zum Anwenden einer Schmerzmodulationsbehandlung optimiert ist, in Übereinstimmung mit zumindest einem Teil der Sensorsignale durchgeführt wird.

21. Verfahren nach einem der Ansprüche 15 bis 20, wobei das Anwenden der Schmerzmodulationsbehandlung eine Behandlung antizipierten Sekundärschmerzes enthält.

22. Verfahren nach einem der Ansprüche 15 bis 21, wobei ein Steuern des Generators ein Einstellen von Parametereinstellungen des Generators zum Einstellen von Parametern der ausgegebenen elektrochirurgischen Energie enthält, wobei Parameter der ausgegebenen elektrochirurgischen Energie eine Pulsfrequenz, eine Wiederholungsrate, eine Wellenform, eine Pulsweite, einen Arbeitszyklus, einen Scheitelfaktor und/oder eine Amplitude enthalten.

23. Verfahren nach einem der Ansprüche 15 bis 22, wobei Schritt (a) durchgeführt wird, bis ein Bereich, der mit der chirurgischen Lokalität verbunden ist, so abgetragen wurde, dass er keine Vaskularität mehr hat, wonach Schritt (b) an der chirurgischen Lokalisation durchgeführt wird, wobei wegen der fehlenden Vaskularität des Bereichs Wärme, die durch das Anwenden der elektrochirurgischen Energie während des Schritts (a) erzeugt wurde, effektiv auf einen Bereich mit einer Vaskularität ausgebreitet wird, der peripher zu dem Bereich ohne Vaskularität ist, um eine Schmerzmodulationsbehandlung für Nerven zur Verfügung zu stellen, die an dem peripheren Bereich mit Vaskularität angeordnet sind.

24. Verfahren nach einem der Ansprüche 15 bis 23, umfassend den Schritt des Steuerns des Generators, um elektrochirurgische Energie auszugeben, um ein elektrochirurgisches Verfahren an einer chirurgischen Lokalität durchzuführen, enthaltend ein Auferlegen eines Arbeitszyklus auf die elektrochirurgische Energie zum Optimieren der ausgegebenen elektrochirurgischen Energie zum Anwenden einer Schmerzmodulationsbehandlung mit einer Veränderung von neuralem Gewebe an einem Ort in und um die chirurgische Lokalisation.

## Revendications

1. Système de commande (18) pour commander un générateur électrochirurgical (16) émettant de l'énergie électrochirurgicale comprenant:
au moins un processeur (102);
un module de commande de procédure (122) pouvant être exécuté sur le au moins un processeur configuré pour produire des signaux de commande pour commander au générateur d'émettre de l'énergie électrochirurgicale qui est optimisée pour l'exécution d'une procédure d'ablation; et **caractérisé par**:
un module de commande de modulation de douleur (122) pouvant être exécuté sur le au moins un processeur configuré pour produire des signaux de commande pour commander au générateur d'émettre de l'énergie électrochirurgicale, ledit module de commande de modulation de la douleur étant optimisé pour administrer un traitement de modulation de la douleur conjointement avec l'exécution de la procédure d'ablation, où le module de commande de modulation de la douleur est configuré pour produire des signaux de commande pour commander au générateur d'émettre de l'énergie électrochirurgicale qui sont prioritaires ou modifient les signaux de commande produits par le module de commande de procédure.

2. Système de commande selon la revendication 1, configuré de façon que le module de commande de modulation de la douleur commande au générateur d'imposer un cycle de service à l'énergie électrochirurgicale pour établir un train d'impulsions associé à l'énergie effective de l'énergie électrochirurgicale émise.

3. Système de commande selon la revendication 1, configuré de façon que la commande du générateur par le module de commande de modulation de la douleur conjointement avec la performance de la procédure électrochirurgicale comprend la commande du générateur pour qu'il émette de l'énergie électrochirurgicale optimisée pour l'administration d'un traitement de modulation de la douleur à au moins un avant l'administration de l'énergie électrochirurgicale pour exécuter la procédure électrochirurgicale, durant l'administration de l'énergie électrochirurgicale pour exécuter la procédure électrochirurgicale et après l'administration de l'énergie électrochirurgicale pour exécuter la procédure électrochirurgicale.

4. Système de commande selon la revendication 3, configuré de façon que:
la commande du générateur comprend l'ajustement de réglages de paramètres du générateur; et
la commande du générateur pour l'émission d'énergie électrochirurgicale optimisée pour administrer le traitement de modulation de la douleur durant l'administration de l'énergie électrochirurgicale pour exécuter la procédure électrochirurgicale comprend au moins une de:
la commande du générateur par le module de commande de modulation de la douleur pour émettre de l'énergie électrochirurgicale qui est optimisée pour administrer un traitement de modulation de la douleur par intermittence avec la commande du générateur par le module de commande de procédure pour émettre de l'énergie électrochirurgicale qui est optimisée pour l'administration durant l'exécution d'une procédure électrochirurgicale; et
la commande du générateur par le module de commande de modulation de la douleur et le module de commande de procédure sensiblement simultanément incluant l'ajustement des réglages des paramètres du générateur en accord avec au moins une fonction pour combiner l'ajustement des réglages de paramètres du générateur pour l'émission d'énergie électrochirurgicale qui est optimisée pour l'administration durant l'exécution d'une procédure électrochirurgicale avec l'ajustement des réglages de paramètres du générateur pour émettre l'énergie électrochirurgicale qui est optimisée pour administrer le traitement de modulation de la douleur.

5. Système de commande selon la revendication 1, 2, 3 ou 4, configuré de façon que le au moins un processeur fonctionne en un mode pouvant être sélectionné parmi chacun d'un mode électrochirurgical dans lequel le générateur est commandé par le module de commande de procédure; un mode de modulation de la douleur dans lequel le générateur est commandé par le module de commande de modulation de la douleur; et un mode de combinaison dans lequel le générateur est commandé à la fois par le module de commande de procédure et le module de commande de la modulation de la douleur.

6. Système de commande selon l'une quelconque des revendications précédentes, configuré de façon que
la commande du générateur comprend l'ajustement des réglages de paramètres du générateur;
le au moins un processeur reçoit des données d'entrée pour au moins un parmi la sélection du mode, l'entrée de données pré-chirurgicales, l'entrée de données d'observation et la sélection de réglages de paramètres du générateur; et
au moins une portion des données d'entrée est utilisée pour déterminer des ajustements aux réglages de paramètres du générateur.

7. Système de commande selon l'une quelconque des revendications précédentes, comprenant en outre un module de détection ayant au moins un capteur détectant des propriétés à ou à proximité d'un site chirurgical ou d'un site lié au site chirurgical et produisant des signaux de détection correspondants, où le module de commande de modulation de la douleur est configuré pour commander le générateur en accord avec au moins une portion des signaux de détection.

8. Système de commande selon l'une quelconque des revendications précédentes, dans lequel le traitement de modulation de la douleur est configuré pour traiter un site éloigné d'un site chirurgical auquel l'énergie électrochirurgicale est administrée durant l'exécution de la procédure électrochirurgicale.

9. Système de commande selon l'une quelconque des revendications précédentes, dans lequel le module de commande de la modulation de la douleur est configuré de façon qu'un traitement de modulation de la douleur comprend le traitement d'une douleur secondaire anticipée.

10. Système de commande selon la revendication 9, ledit traitement de la douleur secondaire étant sélectionné dans le groupe consistant en laser, cryogénique, micro-ondes, ultrasons haute intensité et ultrasons.

11. Système de commande selon l'une quelconque des revendications précédentes, configuré de façon que la commande du générateur comprend l'ajustement de réglages de paramètres du générateur pour ajuster les paramètres de l'énergie électrochirurgicale émise, où les paramètres de l'énergie électrochirurgicale émise comprennent au moins une d'une fréquence d'impulsion, fréquence de porteuse, configuration de forme d'onde, largeur d'impulsion, cycle de service, facteur de crête, amplitude, modulation d'amplitude, un recouvrement de traitement et une modulation de fréquence.

12. Système de commande selon la revendication 3 ou l'une quelconque des revendications dépendant de celle-ci, configuré de façon que le mode de modulation de la douleur comprend en outre un mode de stimulation pouvant être sélectionné ayant au moins une de fonctions sensorielle et motrice pour déterminer la proximité d'un nerf ou d'un paquet de nerfs.

13. Système de commande selon l'une quelconque des revendications précédentes pour commander un générateur radio-fréquence (RF) émettant une énergie RF.

14. Système de commande selon l'une quelconque des revendications précédentes, dans lequel ledit traitement de commande de la modulation de la douleur convient pour moduler ladite sortie pour stimuler un tissu afin de réduire une quantité d'un mécanisme douloureux.

15. Procédé de commande d'un générateur électrochirurgical (16) émettant de l'énergie électrochirurgicale, le procédé comprenant les étapes de:
(a) produire des signaux de commande, en commandant ainsi le générateur pour qu'il émette l'énergie électrochirurgicale qui est optimisée pour exécuter une procédure d'ablation; et **caractérisé par**:
(b) produire des signaux de commande, en commandant ainsi au générateur d'émettre de l'énergie électrochirurgicale qui est optimisée pour administer un traitement de modulation de la douleur conjointement avec l'exécution de la procédure d'ablation,
où les signaux de commande produits à l'étape (b) sont prioritaires ou modifient les signaux de commande produits à l'étape (a).

16. Procédé selon la revendication 15, dans lequel l'étape (b) comprend la commande du générateur pour imposer un cycle de service à l'énergie électrochirurgicale pour établir un train d'impulsions associé à l'énergie effective de l'énergie électrochirurgicale de sortie.

17. Procédé selon la revendication 15, dans lequel l'étape (b) comprend la commande du générateur pour qu'il émette de l'énergie électrochirurgicale optimisée pour l'administration d'un traitement de modulation de la douleur au moins à un parmi avant l'administration de l'énergie électrochirurgicale pour exécuter la procédure électrochirurgicale, durant l'administration de l'énergie électrochirurgicale pour exécuter la procédure électrochirurgicale et après l'administration de l'énergie électrochirurgicale pour exécuter la procédure électrochirurgicale.

18. Procédé selon la revendication 17, dans lequel:
la commande du générateur comprend l'ajustement de réglages de paramètres du générateur; et
la commande du générateur pour qu'il émette de l'énergie électrochirurgicale optimisée pour administrer un traitement de modulation de la douleur durant l'administration de l'énergie électrochirurgicale pour exécuter la procédure électrochirurgicale comprend au moins une de:
commander au générateur d'émettre de l'énergie électrochirurgicale qui est optimisée pour administrer un traitement de modulation de la douleur par intermittence en commandant au générateur d'émettre de l'énergie électrochirurgicale qui est optimisée pour l'administration durant l'exécution d'une procédure électrochirurgicale; et
commander au générateur d'émettre de l'énergie électrochirurgicale optimisée à la fois pour l'administration du traitement de modulation de la douleur et pour l'exécution de la procédure électrochirurgicale sensiblement simultanément, comprenant l'ajustement des réglages des paramètres du générateur en accord avec au moins une fonction pour combiner l'ajustement des réglages des paramètres du générateur pour émettre de l'énergie électrochirurgicale qui est optimisée pour l'administration durant l'exécution d'une procédure chirurgicale et l'ajustement de réglages de paramètres du générateur pour émettre de l'énergie électrochirurgicale qui est optimisée pour l'administration du traitement de modulation de la douleur.

19. Procédé selon l'une quelconque des revendications 15 à 18, dans lequel la commande de l'étape de générateur comprend
l'ajustement des réglages des paramètres du générateur;
la réception de données d'entrée pour au moins une consistant à entrer des données préchirurgicales, entrer des données d'observation et sélectionner des réglages de paramètres du générateur; et
ajuster les réglages des paramètres du générateur en utilisant au moins une partie des données d'entrée pour déterminer l'ajustement.

20. Procédé selon l'une quelconque des revendications 15 à 19, le procédé comprenant en outre les étapes de:
recevoir des signaux de détection correspondant à la détection de propriétés à ou à proximité du site chirurgical ou d'un site lié au site chirurgical;
où la commande du générateur pour qu'il émette de l'énergie électrochirurgicale optimisée pour l'administration du traitement de modulation de la douleur est exécutée en accord avec au moins une partie des signaux de détection.

21. Procédé selon l'une quelconque des revendications 15 à 20, dans lequel l'administration du traitement de modulation de la douleur comprend le traitement d'une douleur secondaire anticipée.

22. Procédé selon l'une quelconque des revendications 15 à 21, dans lequel la commande du générateur comprend l'ajustement des réglages des paramètres du générateur pour ajuster les paramètres de l'énergie chirurgicale de sortie, où les paramètres de l'énergie électrochirurgicale de sortie comprennent au moins un parmi la fréquence d'impulsions, le taux de répétition, la configuration de la forme d'onde, la largeur d'impulsion, le cycle de service, le facteur de crête et l'amplitude.

23. Procédé selon l'une quelconque des revendications 15 à 22, dans lequel l'étape (a) est exécutée jusqu'à ce qu'une zone associée au site chirurgical soit retirée par ablation de sorte qu'elle n'a plus de vascularité, après quoi l'étape (b) est exécutée au site chirurgical, où dû à l'absence de la vascularité de la zone, la chaleur provoquée par l'administration de l'énergie électrochirurgicale durant l'étape (a) est étalée efficacement à une zone ayant une vascularité qui est périphérique à la zone exempte de vascularité pour fournir un traitement de modulation de la douleur aux nerfs situés à la zone périphérique ayant une vascularité.

24. Procédé selon l'une quelconque des revendications 15 à 23, comprenant l'étape consistant à commander au générateur d'émettre de l'énergie électrochirurgicale pour exécuter une procédure électrochirurgicale à un site chirurgical incluant l'imposition d'un cycle de service à l'énergie électrochirurgicale pour optimiser l'énergie électrochirurgicale émise pour l'administration d'un traitement de modulation de la douleur incluant la modification du tissu neural à un emplacement dans et autour du site chirurgical.
